# EUROPEAN PATENT SPECIFICATION

(11) **EP 2 544 537 B1**
(45) Date of publication and mention of the grant of the patent: **10.05.2017**
(21) Application number: 11754115.1
(22) Date of filing: 10.03.2011
(51) Int. Cl.: A01N 43/02, A61K 31/335, A61K 9/00, A61K 47/12, A61K 47/26, A61K 9/19

(54) **PARENTERAL FORMULATIONS OF MACROLIDE ANTIBIOTICS**
PARENTERALE FORMULIERUNGEN AUS MAKROLIDANTIBIOTIKA
FORMULATIONS PARENTÉRALES D'ANTIBIOTIQUES DE LA CLASSE DES MACROLIDES

(30) Priority: 10.03.2010 US 312417 P
(43) Date of publication of application: 16.01.2013
(73) Proprietor: Cempra Pharmaceuticals, Inc., Chapel Hill, NC 27517 (US)
(72) Inventor: PEREIRA, David, E., Apex NC 27502 (US); FERNANDES, Prabhavathi, Chapel Hill NC 27514 (US)
(74) Representative: Elsy, David
(86) International application number: PCT/US2011/027984
(87) International publication number: WO 2011/112864

(56) References cited:
- WO-A1-03/072141
- US-A1- 2006 100 164
- US-A1- 2006 100 164
- US-A1- 2008 221 048
- US-A1- 2009 156 517
- DATABASE WPI Week 200822 Thomson Scientific, London, GB; AN 2008-D02982 XP002722744, & CN 101 045 063 A (GUANGZHOU LANGSHENG PHARMACY CO LTD) 3 October 2007 (2007-10-03)

## Description

### TECHNICAL FIELD

The invention described herein pertains to pharmaceutical compositions adapted for the parenteral administration of macrolide antibiotics, such as triazole-containing and fluoroketolide antibiotics. The invention described herein also pertains to methods for their use in the treatment of bacterial, protozoal, and other infections.

### BACKGROUND AND SUMMARY OF THE INVENTION

Macrolide antibiotics, characterized by a large lactone ring to which are attached one or more deoxy sugars, usually cladinose and desosamine, are antimicrobial drugs that are active against aerobic and anaerobic gram positive cocci and are prescribed for the treatment of a number of infections, including respiratory tract and soft tissue infections. The macrolides, which belong to the polyketide class of natural products, function by reversibly binding to the 50S subunit of the bacterial ribosome, blocking protein synthesis and preventing bacterial growth and reproduction. Although this action is primarily bacteriostatic, certain fluoroketolides triazole-containing macrolides are bactericidal. Other macrolides may be bactericidal at higher concentrations.

Ketolides, which are semi-synthetic derivatives of the 14-membered macrolide erythromycin A, belong to the class of drugs used to treat respiratory tract infections. These drugs are effective against macrolide-resistant bacteria because of their ability to bind to two sites on the bacterial ribosome. Even so, acquired bacterial resistance to macrolides may occur, such as by post-transcriptional methylation of the 23S bacterial ribosome. This resistance results in cross-resistance to macrolides, lincosamides and streptogramins. Although rare, acquired resistance also can result from the production of drug-inactivating enzymes such as esterases or kinases, as well as the production of active ATP-dependent efflux proteins that transport macrolides out of the cell. A significant fraction of pneumococci are resistant to currently available antibiotics.

Erythromycin and the semi-synthetic derivatives azithromycin and clarithromycin are among the marketed macrolide antibiotics. Telithromycin and cethromycin belong to the ketolide group of antibiotics. Oral administration has been accomplished for many macrolides and ketolides, including erythromycin, clarithromycin, telithromycin, and azithromycin. However, the corresponding parenteral administration, such as intravenous (IV) and intramuscular (IM) administration of known macrolides and ketolides, especially approved macrolides such as erythromycin, clarithromycin, telithromycin, and azithromycin, has been hampered by pharmacologic pain upon administration, the observation of QT prolongation, hepatoxicity, inflammation, and other adverse events. For example, erythromycin, clarithromycin, and azithromycin have been reported to be painful when administered parenterally, leading to limitations on their use, issues with patient compliance, and other disadvantages. In addition, clarithromycin and telithromycin have been reported to result in unacceptable QT prolongation when administered parenterally. Without being bound by theory, it is believed herein that the pharmacokinetic (PK) and/or pharmacodynamic (PD) behavior associated with the parenteral administration of approved macrolides causes and/or exacerbates one or both of these adverse events. In addition, but without being bound by theory, it is believed herein that the reported preferential tissue distribution of parenterally administered macrolides to cardiac tissue may also cause and/or exacerbate the QT prolongation.

Currently, only clarithromycin, and azithromycin. two macrolides, are approved for intravenous (IV) administration outside the United States, where only azithromycin is approved in the United States. Currently, there are no ketolides approved for parenteral administration anywhere in the world. Even though approved, the use of IV administered clarithromycin and azithromycin may be severely limited due to the foregoing adverse event observations. Accordingly, a need exists for alternative parenteral formulations of macrolides, especially ketolides to address the ever-present issues of current and developing resistance, and methods for using such parenteral formulations in the treatment of bacterial, protozoal, and other infections. Provided herein are pharmaceutical compositions adapted for the parenteral administration of the triazole-containing and fluoroketolide antibiotics, such as CEM-101 and related compounds, as well as methods for their use in the treatment of bacterial, protozoal, and other infections.

It has been discovered herein that triazole-containing and fluoroketolide antibiotics, such as CEM-101 and related compounds, may be formulated for parenteral administration, including IV and IM administration. It has also been discovered herein that triazole-containing and fluoroketolide antibiotics, such as CEM-101 and related compounds, do not cause pharmacologic pain upon injection. Further, it has been discovered herein that triazole-containing and fluoroketolide antibiotics, such as CEM-101 and related compounds, do not accumulate in cardiac tissue, and do not cause QT prologation.

In international patent application, publication number WO 2004/080391, there are disclosed a family of triazole-containing and fluoroketolide antibiotics. Illustrative of those antibiotics are compounds of the formula: 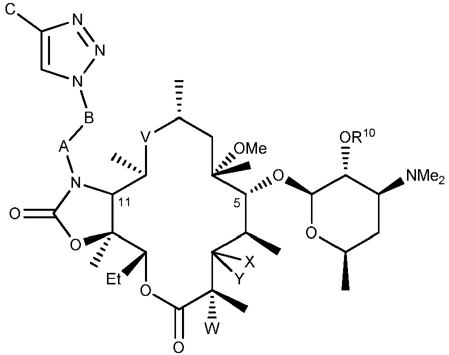 and pharmaceutically acceptable salts, hydrates, solvates, esters, and prodrugs thereof, wherein:
R₁₀ is hydrogen or acyl;
X is H; and Y is OR₇; where R₇ is a monosaccharide or disaccharide, alkyl, aryl, heteroaryl, acyl, or C(O)NR₈R₉, where R₈ and R₉ are each independently selected from the group consisting of hydrogen, hydroxy, alkyl, aralkyl, alkylaryl, heteroalkyl, aryl, heteroaryl, alkoxy, dimethylaminoalkyl, acyl, sulfonyl, ureido, and carbamoyl; or X and Y are taken together with the attached carbon to form carbonyl;
V is C(O), C(=NR₁₁), CH(NR₁₂, R₁₃), or N(R₁₄)CH₂, where N(R₁₄) is attached to the C-10 carbon of the compound; wherein R₁₁ is hydroxy or alkoxy, R₁₂ and R₁₃ are each independently selected from the group consisting of hydrogen, hydroxy, akyl, aralkyl, alkylaryl, alkoxy, heteroalkyl, aryl, heteroaryl, dimethylaminoalkyl, acyl, sulfonyl, ureido, and carbamoyl; R₁₄ is hydrogen, hydroxy, alkyl, aralkyl, alkylaryl, alkoxy, heteroalkyl, aryl, heteroaryl, dimethylaminoalkyl, acyl, sulfonyl, ureido, or carbamoyl;
W is H, F, Cl, Br, I, or OH;
A is CH₂, C(O), C(O)O, C(O)NH, S(O)₂, S(O)₂NH, C(O)NHS(O)₂;
B is (CH₂)ₙ where n is an integer ranging from 0-10, or B is an unsaturated carbon chain of 2-10 carbons; and
C is hydrogen, hydroxy, alkyl, aralkyl, alkylaryl, alkoxy, heteroalkyl, aryl, heteroaryl, aminoaryl, alkylaminoaryl, acyl, acyloxy, sulfonyl, ureido, or carbamoyl, each of which is optionally substituted.

Further illustrative of those compounds is CEM-101, Chemical Abstracts Registry Number 760981-83-7, and having the following structure: 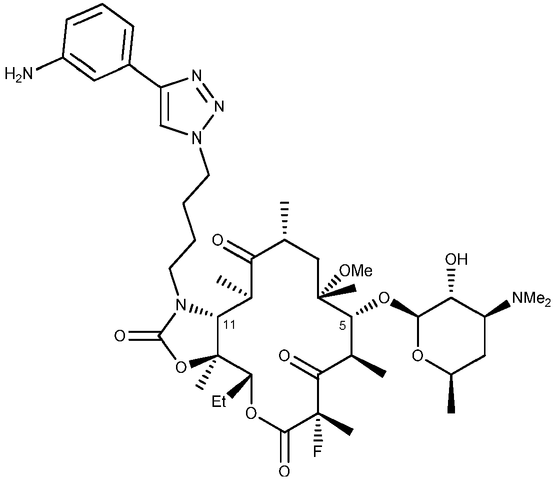 and pharmaceutically acceptable salts, hydrates, solvates, esters, and prodrugs thereof.

### DETAILED DESCRIPTION

CEM-101 and related compounds are highly potent macrolides that retain activity against drug-resistant strains, including showing potent activity against S. *pneumoniae,* as well as having an extended spectrum of activity against community acquired-methicillin resistant *Staphylococcus aureus* (CA-MRSA), *enterococci, M. avium,* and showing efficacy in animal models of malaria. They are also active against atypical bacteria, such as *Leginella, Mycoplasma* and *Ureaplasma,* and against gonococci and other organisms that cause genitouriniary tract infections. Illustratively, CEM-101 is 8-16 times more potent than azithromycin and is active against azithromycin-resistant strains. Without being bound by theory, it is believed herein that the activity of CEM-101 and related compounds against resistant strains may be driven by their ability to bind to three sites on the bacterial ribosome, compared to one or two sites for currently available macrolides. There is provided a pharmaceutical composition adapted for parenteral administration comprising CEM-101 (solithromycin) or a pharmaceutically acceptable salt thereof; and an acidifying agent comprising a tartaric acid or citric acid, or a combination thereof; where the composition is capable of reconstitution in one or more aqueous diluents.

There is also provided a lyophilized pharmaceutical composition, adapted for dilution to a pharmaceutical composition for parenteral administration, comprising CEM-101 (solithromycin) or a pharmaceutically acceptable salt thereof, an acidifying agent comprising a tartaric acid or citric acid, or a combination thereof, an alkalizing agent, and at least one additional excipient.

The antibiotic compound CEM-101 or a related compound as described above may be prepared as described in WO 2004/080391 or WO 2009/055557, or by conventional procedures, or by a procedure analogous to one of the described or known procedures.

In another embodiment, the compound is CEM-101, or one or more pharmaceutically acceptable salt thereof.

In another embodiment, the composition is one wherein the excipients include, but are not limited to, mannitol, sucrose, glycine, and combinations thereof. The acidifying agent is citric acid, and/or a tartaric acid, or a combination thereof. In another embodiment, the acidifying agent is a tartaric acid, such as L-tartaric acid.

In another embodiment of the composition, the concentration of the acidifying agent is about 30 mM to about 70 mM. In another embodiment of the composition, the concentration of the acidifying agent is about 50 mM to about 60 mM. In a further embodiment of the composition, the concentration of the acidifying agent is about 60 mM. In another embodiment of the composition, the ratio of CEM-101 or related compound to the acidifying agent is in the range from about 100:1 to about 2:1. In another embodiment of the composition, the ratio of CEM-101 or related compound to the acidifying agent is in the range from about 20:1 to about 5:1. In each of the foregoing embodiments, the composition may be used as the concentrate described hrein, oir alternatively, may be used as a dried form of the composition, such as a lyophilized, freeze-dried, or spray-dried powder.

Another embodiment of the composition herein is one comprising on a per cent weight to weight or weight to volume basis, a compound described herein such as CEM-101 (about 5%), L-(+)-tartaric acid (about 0.6%), sodium hydroxide (about 0.05%), and water for injection (to 100%). Another embodiment of the composition herein is one comprising on a per cent weight to weight or weight to volume basis, a compound described herein such as CEM-101 (5.0%), L-(+)-tartaric acid (0.58%), sodium hydroxide (0.046%), and water for injection (to 100%). In one variation, the composition is a concentrate that is administered, or alternatively that is adaptable for dilution prior to administration. In another variation, the composition is a dried residue or solid adaptable for reconstitution as a concentrate that is administered, or alternatively that is adaptable for dilution prior to administration. In another variation, the composition is a dried residue or solid adaptable for reconstitution in a diluent for administration.

Another embodiment of the composition herein is one comprising on a per cent weight to weight or weight to volume basis, a compound described herein such as CEM-101 (about 5%), L-(+)-tartaric acid (about 0.6%), sodium hydroxide (about 0.05%), 1-thioglycerol (about 0.5%), and water for injection (to 100%). A further embodiment of the composition herein is one comprising on a per cent weight to weight or weight to volume basis, a compound described herein such as CEM-101 (5.0%), L-(+)-tartaric acid (0.58%), sodium hydroxide (0.046%), 1-thioglycerol (0.50%), and water for injection (to 100%). In one variation, the composition is a concentrate that is administered, or alternatively that is adaptable for dilution prior to administration. In another variation, the composition is a dried residue or solid adaptable for reconstitution as a concentrate that is administered, or alternatively that is adaptable for dilution prior to administration. In another variation, the composition is a dried residue or solid adaptable for reconstitution in a diluent for administration.

In another embodiment of the composition herein is one comprising on a per cent weight to weight or weight to volume basis, a compound described herein such as CEM-101 (about 5%), L-(+)-tartaric acid (about 0.6%), sodium hydroxide (about 0.05%), mannitol (about 3-20%), and water for injection (to 100%). Another embodiment of the composition herein is one comprising on a per cent weight to weight or weight to volume basis, a compound described herein such as CEM-101 (5.0%), L-(+)-tartaric acid (0.58%), sodium hydroxide (0.046%), mannitol (5.0%), and water for injection (to 100%). Another embodiment of the composition herein is one comprising on a per cent weight to weight or weight to volume basis, a compound described herein such as CEM-101 (5.0%), L-(+)-tartaric acid (0.58%), sodium hydroxide (0.046%), mannitol (10%), and water for injection (to 100%). In one variation, the composition is a concentrate that is administered, or alternatively that is adaptable for dilution prior to administration. In another variation, the composition is a dried residue or solid adaptable for reconstitution as a concentrate that is administered, or alternatively that is adaptable for dilution prior to administration. In another variation, the composition is a dried residue or solid adaptable for reconstitution in a diluent for administration.

In another embodiment of the composition herein is one comprising on a per cent weight to weight or weight to volume basis, a compound described herein such as CEM-101 (about 5%), L-(+)-tartaric acid (about 0.6%), sodium hydroxide (about 0.05%), mannitol (about 3-20%), 1-thioglycerol (about 0.5%), and water for injection (to 100%). A further embodiment of the composition herein is one comprising on a per cent weight to weight or weight to volume basis, a compound described herein such as CEM-101 (5.0%), L-(+)-tartaric acid (0.58%), sodium hydroxide (0.046%), mannitol (5.0%), 1-thioglycerol (0.50%), and water for injection (to 100%). A further embodiment of the composition herein is one comprising on a per cent weight to weight or weight to volume basis, a compound described herein such as CEM-101 (5.0%), L-(+)-tartaric acid (0.58%), sodium hydroxide (0.046%), mannitol (10%), 1-thioglycerol (0.50%), and water for injection (to 100%). In one variation, the composition is a concentrate that is administered, or alternatively that is adaptable for dilution prior to administration. In another variation, the composition is a dried residue or solid adaptable for reconstitution as a concentrate that is administered, or alternatively that is adaptable for dilution prior to administration. In another variation, the composition is a dried residue or solid adaptable for reconstitution in a diluent for administration.

In another embodiment, the composition herein is one comprising about 50 mg/mL of a compound described herein such as CEM-101, about 50, about 100, or about 200 mg/mL of one or more excipents, where the excipients are selected from mannitol, glycine, sucrose, and combinations thereof, about 6 mg/mL of a tartartic acid, such as L-(+)-tartaric acid, about 0.5 mg/mL sodium hydroxide, and sterile water for injection. In one variation, the total volume is 1 mL, 2 mL, 4 mL, 8 mL, or 16 mL.

In each of the foregoing embodiments, lyophilized formulations may be prepared. Lyophilized formulations may be reconstituted, such as by dissolution, in any of a wide variety of IV solutions for administration, including but not limited to SWFI, PBS, such as physiological PBS, saline, such as physiological saline, 1N NaCl, 0.5 N NaCl, and he like, D5W, Ringer's, and the like.

Another embodiment of the composition is one further comprising an alkalizing agent. In one embodiment, the alkalizing agent is sodium hydroxide. In one embodiment, the pH of the composition is not less than 2.5. In another embodiment, the pH of the composition is between about 3.7 and about 4.4. In a further embodiment, the pH of the composition is between about 3.8 and about 4.2. In a further embodiment, the pH of the composition is about 4. It is to be understood that the relative amount of alkalizing agent may dependent upon the amount of acidifying agent, or ratio of CEM-101 or related compound to the acidifying agent.

One embodiment of the composition herein is one wherein the concentration of CEM-101 is at least about 5 mg/mL. In another embodiment of the composition herein is one wherein the concentration of CEM-101 is at least about 10 mg/mL. In another embodiment of the composition herein is one wherein the concentration of CEM-101 is at least about 25 mg/mL. In another embodiment of the composition herein is one wherein the concentration of CEM-101 is at least about 30 mg/mL. Another embodiment of the composition herein is one wherein the concentration of CEM-101 is at least about 50 mg/mL. A further embodiment of the composition herein is one wherein the concentration of CEM-101 is about 50 mg/mL. In another embodiment of the composition herein, the concentration of CEM-101 or related compound is less than about 100 mg/mL.

In one embodiment of the composition herein, the saturated solubility of CEM-101 is at least about 50 mg/mL. In another embodiment of the composition herein, the saturated solubility of CEM-101 is at least about 80 mg/mL. In another embodiment of the composition herein, the concentration of CEM-101 or related compound is less than about 100 mg/mL.

A further embodiment of the composition herein is one further comprising an anti-oxidant and/or a chelating agent. In one embodiment, the chelating agent is EDTA. In one embodiment, the anti-oxidant is 1-thioglycerol (also referred to as monothioglycerol or MTG). In one embodiment, the concentration of the anti-oxidant is about 5 mg/mL.

It is to be understood that in every case, the above compositions alternatively may be expressed on a per cent weight to weight basis.

Examples of preparations of such pharmaceutical compositions adapted for parenteral administration comprising the antibiotic compound CEM-101 are provided below in the Examples.

In one embodiment, the pharmaceutical composition described herein is administered directly. In another embodiment, the pharmaceutical composition described herein is administered after further dilution. In another embodiment, the pharmaceutical composition described herein is administered after further redissolution, reconstitution, and/or resuspension.

A further embodiment is a single dose or multiple dose pharmaceutical dosage unit comprising a therapeutically effective amount of a pharmaceutical composition adapted for parenteral administration as described herein. In one embodiment, the dosage unit is an ampoule, a vial, a prefilled syringe, or a bag. In one embodiment, the dosage unit is a single dose unit. In another embodiment, the dosage unit is a multiple dose unit.

An additional embodiment is a process for preparing pharmaceutical compositions adapted for parenteral administration as described herein comprising
dissolving the required amounts of tartaric acid and sodium hydroxide in approximately 50-80% of the required water for injection, to form a first solution,
dissolving the required amount of 1-thioglycerol in the above solution to form a further solution,
dissolving the required amount of CEM-101 in the above solution, optionally adding more of the water for injection, and
making up the solution to the final volume with water for injection.
In each of the foregoing, one or more steps are optionally performed under an inert atmosphere, such as nitrogen and/or argon. In each of the foregoing, one or more steps are optionally performed using water for injection that is nitrogen sparged, and/or argon sparged. In another embodiment, the processes described herein include the step of nitrogen sparging and/or nitrogen purging.

In another embodiment, the processes described herein include the step of sterilizing the formulation. Sterilization may be accomplished by any conventional process step, including but not limited to, by autoclaving (terminal sterilization), such as at a temperature of about 100 °C to about 125 °C, or at about 121 °C, by filtration, such as filtration using SUPOR membrane filter (0.2 µm) - Hydrophilic Polyethersulfone, DURAPORE membrane filter (0.22 µm) - Polyvinylidene Fluoride (Hydrophilic), NYLON membrane filter (0.2 µm) - Nylon Hydrophilic, and the like.

In another embodiment, the compositions described herein are solids that may be redissolved, reconstituted, or otherwise resuspended prior to use to prepare compositions for parenteral administration. Illustratively, the solid is a powder, semicrystalline, or crystalline material prepared by lyophilization, freeze-drying, spray drying, and the like. The solids include CEM-101 and/or related compounds, including fluoroketolines, triazole-containing macrolides, triazole containing ketolides, and triazole containing fluoroketolides, and optionally one or more acidifying agents, alkalizing agents, and/or excipients, such as one or more bulking agents. It is appreciated herein that the solids may be prepared from the solutions of CEM-101 and/or related compounds, acidifying agents, and diluents. It is further appreciated that the solids may be prepared from the solutions of CEM-101 and/or related compounds, acidifying agents, alkalizing agents, and diluents. It is further appreciated that the solids may be prepared from the solutions of CEM-101 and/or related compounds, acidifying agents, alkalizing agents, bulking agents, and diluents. Without being bound by theory, it is believed herein that the one or more bulking agents may at least partially contribute to the characteristics of the resulting solid that provide for rapid redissolution, reconstitution, or resuspension. Without being bound by theory, it is understood herein that the excipients may contribute to the physiologically acceptable osmolality of the formulations.

In another embodiment, the compositions described herein include a bulking agent. Illustrative bulking agents include sugars or carbohydrates, such as mannitol, sucrose, and the like, amino acids, such as glycine, and the like, and combinations thereof. In another embodiment, the ratio of bulking agent to the CEM-101 or related compound is in the range from about 1:2 to about 10:1. In another embodiment, the ratio of bulking agent to the CEM-101 or related compound is in the range from about 1:1 to about 5:1. In another embodiment, the ratio of bulking agent to the CEM-101 or related compound is in the range from about 1:1 to about 4:1.

Another embodiment is a lyophilized pharmaceutical composition, adapted for dilution to afford a pharmaceutical composition for parenteral administration, comprising CEM-101 or a related compound, an acidifying agent, an alkalizing agent, and at least one additional excipient. Inter alia, the excipient may function as a bulking agent, a tonicity adjusting agent, a stabilizing agent, a buffer, an antioxidant and/or a cryoprotectant.

In one embodiment of the lyophilized composition, the acidifying agent is ascorbic acid, citric acid or a tartaric acid. In another embodiment, the acidifying agent is L-tartaric acid. In one embodiment, the L-tartaric acid is present at a ratio to the CEM-101 or a related compound in the range from about 0.01:1 to about 0.5:1.

In one embodiment of the lyophilized composition, the alkalizing agent is sodium hydroxide.

One embodiment of the lyophilized composition comprises the excipient glycine, sucrose, or mannitol, or a related bulking agent. In another embodiment, the bulking agent, such as mannitol, is present at a ratio to the CEM-101 or a related compound in the range from about 0.5:1 to about 5:1. In another embodiment, the bulking agent is mannitol present at a ratio to the CEM-101 or a related compound in the range from about 1:1 to about 4:1. In another embodiment, the bulking agent is glycine present at a ratio to the CEM-101 or a related compound in the range from about 1:1 to about 4:1. In another embodiment, the bulking agent is sucrose present at a ratio to the CEM-101 or a related compound in the range from about 1:1 to about 4:1.

In another embodiment, the compositions include a stabilizing agent. Illustrative stabilizing agents include antioxidants, chelating agents, and the like, such as but not limited to ascorbic acid, cysteine, ethylenediaminetetraacetic acid (EDTA), glutathione, 1-thioglycerol, sodium bisulphite, sodium metabisulphite, and the like. Illustrative concentrations of stabilizers, including anti-oxidants include, but are not limited to, 0.05%, 0.15%, 0.25%, 0.5% and 1.0%, and the like. Illustrative levels of anti-oxidants excluding EDTA include, but are not limited to, 0.25%, 0.5% and 1.0%, and the like. Illustrative levels of EDTA include, but are not limited to, 0.05%, 0.15% and 0.25%.

A further embodiment comprises a single dose or multiple dose pharmaceutical dosage unit comprising a therapeutically effective amount of a lyophilized pharmaceutical composition as described herein. In one embodiment, the dosage unit is an ampoule or a vial. In one embodiment, the dosage unit is a single dose unit. In one embodiment, the dosage unit is a multiple dose unit.

A further embodiment comprises a kit, comprising a pharmaceutical dosage unit comprising a therapeutically effective amount of a lyophilized composition as described herein, and optionally further comprising a vehicle for dilution of the pharmaceutical composition. In another aspect, the kit may include instructions for use. In one illustrative kit, the CEM-101 or related compound is present as a single dose, or multiple dose concentrate. It is appreciated that the concentrate may be administered directly, or alternatively is further diluted into a diluent for administration, such as a 500 mL IV bag containing a suitable carrier, such as 0.5 N or 1 N saline, D5W, Ringer's solution, PBS, and the like, as described herein.

In another illustrative kit, the CEM-101 or related compound is present as a single dose, or multiple dose solid. In one variation, the kit also includes a reconstitution solution. It is appreciated that the reconstitution solution may be for the purpose of preparing a concentrate that is administered directly, or alternatively is further diluted into a diluent for administration, such as a 500 mL IV bag containing a suitable carrier, such as 0.5 N or 1 N saline, D5W, Ringer's solution, PBS, and the like, as described herein.

It will be understood that in the description and claims herein that CEM-101, which is basic, and related compounds, which are basic, will be present in protonated form in solutions containing an acid. Accordingly, CEM-101 and related compounds, denote not only the free base, but also the protonated form in the context of a pharmaceutical composition adapted for parenteral administration. It will be understood that the acidifying agent and the alkalizing agent as described herein, when used together in an aqueous system, form a buffering agent, and may alternatively be denoted as such, for example as a L-tartaric acid/NaOH buffer, citric acid/NaOH buffer, and the like.

As used herein, the term "alkyl" includes a chain of carbon atoms, which is optionally branched. As used herein, the term "alkenyl" and "alkynyl" includes a chain of carbon atoms, which is optionally branched, and includes at least one double bond or triple bond, respectively. It is to be understood that alkynyl may also include one or more double bonds. It is to be further understood that in certain embodiments, alkyl is advantageously of limited length, including C₁-C₂₄, C₁-C₁₂, C₁-C₈, C₁-C₆, and C₁-C₄. It is to be further understood that in certain embodiments alkenyl and/or alkynyl may each be advantageously of limited length, including C₂-C₂₄, C₂-C₁₂, C₂-C₈, C₂-C₆, and C₂-C₄. It is appreciated herein that shorter alkyl, alkenyl, and/or alkynyl groups may add less lipophilicity to the compound and accordingly will have different pharmacokinetic behavior. Illustrative alkyl groups are, but not limited to, methyl, ethyl, n-propyl, isopropyl, n-butyl, isobutyl, sec-butyl, tert-butyl, pentyl, 2-pentyl, 3-pentyl, neopentyl, hexyl, heptyl, octyl and the like.

As used herein, the term "cycloalkyl" includes a chain of carbon atoms, which is optionally branched, where at least a portion of the chain in cyclic. It is to be understood that cycloalkylalkyl is a subset of cycloalkyl. It is to be understood that cycloalkyl may be polycyclic. Illustrative cycloalkyl include, but are not limited to, cyclopropyl, cyclopentyl, cyclohexyl, 2-methylcyclopropyl, cyclopentyleth-2-yl, adamantyl, and the like. As used herein, the term "cycloalkenyl" includes a chain of carbon atoms, which is optionally branched, and includes at least one double bond, where at least a portion of the chain in cyclic. It is to be understood that the one or more double bonds may be in the cyclic portion of cycloalkenyl and/or the non-cyclic portion of cycloalkenyl. It is to be understood that cycloalkenylalkyl and cycloalkylalkenyl are each subsets of cycloalkenyl. It is to be understood that cycloalkyl may be polycyclic. Illustrative cycloalkenyl include, but are not limited to, cyclopentenyl, cyclohexylethen-2-yl, cycloheptenylpropenyl, and the like. It is to be further understood that chain forming cycloalkyl and/or cycloalkenyl is advantageously of limited length, including C₃-C₂₄, C₃-C₁₂, C₃-C₈, C₃-C₆, and C₅-C₆. It is appreciated herein that shorter alkyl and/or alkenyl chains forming cycloalkyl and/or cycloalkenyl, respectively, may add less lipophilicity to the compound and accordingly will have different pharmacokinetic behavior.

As used herein, the term "heteroalkyl" includes a chain of atoms that includes both carbon and at least one heteroatom, and is optionally branched. Illustrative heteroatoms include nitrogen, oxygen, and sulfur. In certain variations, illustrative heteroatoms also include phosphorus, and selenium. As used herein, the term "cycloheteroalkyl" including heterocyclyl and heterocycle, includes a chain of atoms that includes both carbon and at least one heteroatom, such as heteroalkyl, and is optionally branched, where at least a portion of the chain is cyclic. Illustrative heteroatoms include nitrogen, oxygen, and sulfur. In certain variations, illustrative heteroatoms also include phosphorus, and selenium. Illustrative cycloheteroalkyl include, but are not limited to, tetrahydrofuryl, pyrrolidinyl, tetrahydropyranyl, piperidinyl, morpholinyl, piperazinyl, homopiperazinyl, quinuclidinyl, and the like.

As used herein, the term "aryl" includes monocyclic and polycyclic aromatic carbocyclic groups, each of which may be optionally substituted. Illustrative aromatic carbocyclic groups described herein include, but are not limited to, phenyl, naphthyl, and the like. As used herein, the term "heteroaryl" includes aromatic heterocyclic groups, each of which may be optionally substituted. Illustrative aromatic heterocyclic groups include, but are not limited to, pyridinyl, pyrimidinyl, pyrazinyl, triazinyl, tetrazinyl, quinolinyl, quinazolinyl, quinoxalinyl, thienyl, pyrazolyl, imidazolyl, oxazolyl, thiazolyl, isoxazolyl, isothiazolyl, oxadiazolyl, thiadiazolyl, triazolyl, benzimidazolyl, benzoxazolyl, benzthiazolyl, benzisoxazolyl, benzisothiazolyl, and the like.

As used herein, the term "amino" includes the group NH₂, alkylamino, and dialkylamino, where the two alkyl groups in dialkylamino may be the same or different, i.e. alkylalkylamino. Illustratively, amino includes methylamino, ethylamino, dimethylamino, methylethylamino, and the like. In addition, it is to be understood that when amino modifies or is modified by another term, such as aminoalkyl, or acylamino, the above variations of the term amino are included therein. Illustratively, aminoalkyl includes H₂N-alkyl, methylaminoalkyl, ethylaminoalkyl, dimethylaminoalkyl, methylethylaminoalkyl, and the like. Illustratively, acylamino includes acylmethylamino, acylethylamino, and the like.

As used herein, the term "amino and derivatives thereof" includes amino as described herein, and alkylamino, alkenylamino, alkynylamino, heteroalkylamino, heteroalkenylamino, heteroalkynylamino, cycloalkylamino, cycloalkenylamino, cycloheteroalkylamino, cycloheteroalkenylamino, arylamino, arylalkylamino, arylalkenylamino, arylalkynylamino, heteroarylamino, heteroarylalkylamino, heteroarylalkenylamino, heteroarylalkynylamino, acylamino, and the like, each of which is optionally substituted. The term "amino derivative" also includes urea, carbamate, and the like.

As used herein, the term "hydroxy and derivatives thereof" includes OH, and alkyloxy, alkenyloxy, alkynyloxy, heteroalkyloxy, heteroalkenyloxy, heteroalkynyloxy, cycloalkyloxy, cycloalkenyloxy, cycloheteroalkyloxy, cycloheteroalkenyloxy, aryloxy, arylalkyloxy, arylalkenyloxy, arylalkynyloxy, heteroaryloxy, heteroarylalkyloxy, heteroarylalkenyloxy, heteroarylalkynyloxy, acyloxy, and the like, each of which is optionally substituted. The term "hydroxy derivative" also includes carbamate, and the like.

As used herein, the term "thio and derivatives thereof" includes SH, and alkylthio, alkenylthio, alkynylthio, heteroalkylthio, heteroalkenylthio, heteroalkynylthio, cycloalkylthio, cycloalkenylthio, cycloheteroalkylthio, cycloheteroalkenylthio, arylthio, arylalkylthio, arylalkenylthio, arylalkynylthio, heteroarylthio, heteroarylalkylthio, heteroarylalkenylthio, heteroarylalkynylthio, acylthio, and the like, each of which is optionally substituted. The term "thio derivative" also includes thiocarbamate, and the like.

As used herein, the term "acyl" includes formyl, and alkylcarbonyl, alkenylcarbonyl, alkynylcarbonyl, heteroalkylcarbonyl, heteroalkenylcarbonyl, heteroalkynylcarbonyl, cycloalkylcarbonyl, cycloalkenylcarbonyl, cycloheteroalkylcarbonyl, cycloheteroalkenylcarbonyl, arylcarbonyl, arylalkylcarbonyl, arylalkenylcarbonyl, arylalkynylcarbonyl, heteroarylcarbonyl, heteroarylalkylcarbonyl, heteroarylalkenylcarbonyl, heteroarylalkynylcarbonyl, acylcarbonyl, and the like, each of which is optionally substituted.

As used herein, the term "carbonyl and derivatives thereof" includes the group C(O), C(S), C(NH) and substituted amino derivatives thereof.

As used herein, the term "sulfonyl or a derivative thereof" includes SO₃H and salts thereof, and esters and amides thereof.

The term "optionally substituted" as used herein includes the replacement of hydrogen atoms with other functional groups on the radical that is optionally substituted. Such other functional groups illustratively include, but are not limited to, amino, hydroxyl, halo, thiol, alkyl, haloalkyl, heteroalkyl, aryl, arylalkyl, arylheteroalkyl, heteroaryl, heteroarylalkyl, heteroarylheteroalkyl, nitro, sulfonic acids and derivatives thereof, carboxylic acids and derivatives thereof, and the like. Illustratively, any of amino, hydroxyl, thiol, alkyl, haloalkyl, heteroalkyl, aryl, arylalkyl, arylheteroalkyl, heteroaryl, heteroarylalkyl, heteroarylheteroalkyl, and/or sulfonic acid is optionally substituted.

As used herein, the terms "optionally substituted aryl" and "optionally substituted heteroaryl" include the replacement of hydrogen atoms with other functional groups on the aryl or heteroaryl that is optionally substituted. Such other functional groups illustratively include, but are not limited to, amino, hydroxy, halo, thio, alkyl, haloalkyl, heteroalkyl, aryl, arylalkyl, arylheteroalkyl, heteroaryl, heteroarylalkyl, heteroarylheteroalkyl, nitro, sulfonic acids and derivatives thereof, carboxylic acids and derivatives thereof, and the like. Illustratively, any of amino, hydroxy, thio, alkyl, haloalkyl, heteroalkyl, aryl, arylalkyl, arylheteroalkyl, heteroaryl, heteroarylalkyl, heteroarylheteroalkyl, and/or sulfonic acid is optionally substituted.

Illustrative substituents include, but are not limited to, a radical -(CH₂)ₓZ^{X}, where x is an integer from 0-6 and Z^{X} is selected from halogen, hydroxy, alkanoyloxy, including C₁-C₆ alkanoyloxy, optionally substituted aroyloxy, alkyl, including C₁-C₆ alkyl, alkoxy, including C₁-C₆ alkoxy, cycloalkyl, including C₃-C₈ cycloalkyl, cycloalkoxy, including C₃-C₈ cycloalkoxy, alkenyl, including C₂-C₆ alkenyl, alkynyl, including C₂-C₆ alkynyl, haloalkyl, including C₁-C₆ haloalkyl, haloalkoxy, including C₁-C₆ haloalkoxy, halocycloalkyl, including C₃-C₈ halocycloalkyl, halocycloalkoxy, including C₃-C₈ halocycloalkoxy, amino, C₁-C₆ alkylamino, (C₁-C₆ alkyl)(C₁-C₆ alkyl)amino, alkylcarbonylamino, N-(C₁-C₆ alkyl)alkylcarbonylamino, aminoalkyl, C₁-C₆ alkylaminoalkyl, (C₁-C₆ alkyl)(C₁-C₆ alkyl)aminoalkyl, alkylcarbonylaminoalkyl, N-(C₁-C₆ alkyl)alkylcarbonylaminoalkyl, cyano, and nitro; or Z^{X} is selected from -CO₂R⁴ and -CONR⁵R⁶, where R⁴, R⁵, and R⁶ are each independently selected in each occurrence from hydrogen, C₁-C₆ alkyl, aryl-C₁-C₆ alkyl, and heteroaryl-C₁-C₆ alkyl.

Monosaccharides, or simple sugars, consist of a single polyhydroxy aldehyde or ketone unit. Representative monosaccharides include, by way of illustration only, hexoses such as D-glucose, D-mannose, D-xylose, D-galactose, L-fucose, and the like; pentoses such as D-ribose or D-arabinose and ketoses such as D-ribulose or D-fructose. Disaccharides contain two monosaccharide units joined by a glycosidic linkage. Disaccharides include, for example, sucrose, lactose, maltose, cellobiose, and the like. Oligosaccharides typically contain from 2 to 10 monosaccharide units joined by glycosidic linkages.

The term "prodrug" as used herein generally refers to any compound that when administered to a biological system generates a biologically active compound as a result of one or more spontaneous chemical reaction(s), enzyme-catalyzed chemical reaction(s), and/or metabolic chemical reaction(s), or a combination thereof. In vivo, the prodrug is typically acted upon by an enzyme (such as esterases, amidases, phosphatases, and the like), simple biological chemistry, or other process in vivo to liberate or regenerate the more pharmacologically active drug. This activation may occur through the action of an endogenous host enzyme or a non-endogenous enzyme that is administered to the host preceding, following, or during administration of the prodrug. Additional details of prodrug use are described in U.S. Pat. No. 5,627,165; and Pathalk et al., Enzymic protecting group techniques in organic synthesis, Stereosel. Biocatal. 775-797 (2000). It is appreciated that the prodrug is advantageously converted to the original drug as soon as the goal, such as targeted delivery, safety, stability, and the like is achieved, followed by the subsequent rapid elimination of the released remains of the group forming the prodrug.

Prodrugs may be prepared from the compounds described herein by attaching groups that ultimately cleave in vivo to one or more functional groups present on the compound, such as -OH-, -SH, -CO₂H, -NR₂. Illustrative prodrugs include but are not limited to carboxylate esters where the group is alkyl, aryl, arylalkyl, heteroaryl, heteroarylalkyl, acyloxyalkyl, alkoxycarbonyloxyalkyl as well as esters of hydroxyl, thiol and amines where the group attached is an acyl group, an alkoxycarbonyl, aminocarbonyl, phosphate or sulfate. Illustrative esters, also referred to as active esters, include but are not limited to 1-indanyl, N-oxysuccinimide; acyloxyalkyl groups such as acetoxymethyl, pivaloyloxymethyl, β-acetoxyethyl, β-pivaloyloxyethyl, 1-(cyclohexylcarbonyloxy)prop-1-yl, (1 -aminoethyl)carbonyloxymethyl, and the like; alkoxycarbonyloxyalkyl groups, such as ethoxycarbonyloxymethyl, α-ethoxycarbonyloxyethyl, β-ethoxycarbonyloxyethyl, and the like; dialkylaminoalkyl groups, including di-lower alkylamino alkyl groups, such as dimethylaminomethyl, dimethylaminoethyl, diethylaminomethyl, diethylaminoethyl, and the like; 2-(alkoxycarbonyl)-2-alkenyl groups such as 2-(isobutoxycarbonyl) pent-2-enyl, 2-(ethoxycarbonyl)but-2-enyl, and the like; and lactone groups such as phthalidyl, dimethoxyphthalidyl, and the like.

Further illustrative prodrugs contain a chemical moiety, such as an amide or phosphorus group functioning to increase solubility and/or stability of the compounds described herein. Further illustrative prodrugs for amino groups include, but are not limited to, (C₃-C₂₀)alkanoyl; halo-(C₃-C₂₀)alkanoyl; (C₃-C₂₀)alkenoyl; (C₄-C₇)cycloalkanoyl; (C₃-C₆)-cycloalkyl(C₂-C₁₆)alkanoyl; optionally substituted aroyl, such as unsubstituted aroyl or aroyl substituted by 1 to 3 substituents selected from the group consisting of halogen, cyano, trifluoromethanesulphonyloxy, (C₁-C₃)alkyl and (C₁-C₃)alkoxy, each of which is optionally further substituted with one or more of 1 to 3 halogen atoms; optionally substituted aryl(C₂-C₁₆)alkanoyl and optionally substituted heteroaryl(C₂-C₁₆)alkanoyl, such as the aryl or heteroaryl radical being unsubstituted or substituted by 1 to 3 substituents selected from the group consisting of halogen, (C₁-C₃)alkyl and (C₁-C₃)alkoxy, each of which is optionally further substituted with 1 to 3 halogen atoms; and optionally substituted heteroarylalkanoyl having one to three heteroatoms selected from O, S and N in the heteroaryl moiety and 2 to 10 carbon atoms in the alkanoyl moiety, such as the heteroaryl radical being unsubstituted or substituted by 1 to 3 substituents selected from the group consisting of halogen, cyano, trifluoromethanesulphonyloxy, (C₁-C₃)alkyl, and (C₁-C₃)alkoxy, each of which is optionally further substituted with 1 to 3 halogen atoms. The groups illustrated are exemplary, not exhaustive, and may be prepared by conventional processes.

It is understood that the prodrugs themselves may not possess significant biological activity, but instead undergo one or more spontaneous chemical reaction(s), enzyme-catalyzed chemical reaction(s), and/or metabolic chemical reaction(s), or a combination thereof after administration in vivo to produce the compound described herein that is biologically active or is a precursor of the biologically active compound. However, it is appreciated that in some cases, the prodrug is biologically active. It is also appreciated that prodrugs may often serves to improve drug efficacy or safety through improved oral bioavailability, pharmacodynamic half-life, and the like. Prodrugs also refer to derivatives of the compounds described herein that include groups that simply mask undesirable drug properties or improve drug delivery. For example, one or more compounds described herein may exhibit an undesirable property that is advantageously blocked or minimized may become pharmacological, pharmaceutical, or pharmacokinetic barriers in clinical drug application, such as low oral drug absorption, lack of site specificity, chemical instability, toxicity, and poor patient acceptance (bad taste, odor, pain at injection site, and the like), and others. It is appreciated herein that a prodrug, or other strategy using reversible derivatives, can be useful in the optimization of the clinical application of a drug.

As used herein, the term "composition" generally refers to any product comprising the specified ingredients in the specified amounts, as well as any product which results, directly or indirectly, from combinations of the specified ingredients in the specified amounts. It is to be understood that the compositions described herein may be prepared from isolated compounds described herein or from salts, solutions, hydrates, solvates, and other forms of the compounds described herein. It is also to be understood that the compositions may be prepared from various amorphous, non-amorphous, partially crystalline, crystalline, and/or other morphological forms of the compounds described herein. It is also to be understood that the compositions may be prepared from various hydrates and/or solvates of the compounds described herein. Accordingly, such pharmaceutical compositions that recite compounds described herein are to be understood to include each of, or any combination of, the various morphological forms and/or solvate or hydrate forms of the compounds described herein. Illustratively, compositions may include one or more carriers, diluents, and/or excipients. The compounds described herein, or compositions containing them, may be formulated in a therapeutically effective amount in any conventional dosage forms appropriate for the methods described herein. The compounds described herein, or compositions containing them, including such formulations, may be administered by a wide variety of conventional routes for the methods described herein, and in a wide variety of dosage formats, utilizing known procedures (see generally, Remington: The Science and Practice of Pharmacy, (21st ed., 2005)).

The term "therapeutically effective amount" as used herein, refers to that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated. In one aspect, the therapeutically effective amount is that which may treat or alleviate the disease or symptoms of the disease at a reasonable benefit/risk ratio applicable to any medical treatment. However, it is to be understood that the total daily usage of the compounds and compositions described herein may be decided by the attending physician within the scope of sound medical judgment. The specific therapeutically-effective dose level for any particular patient will depend upon a variety of factors, including the disorder being treated and the severity of the disorder; activity of the specific compound employed; the specific composition employed; the age, body weight, general health, gender and diet of the patient: the time of administration, route of administration, and rate of excretion of the specific compound employed; the duration of the treatment; drugs used in combination or coincidentally with the specific compound employed; and like factors well known to the researcher, veterinarian, medical doctor or other clinician of ordinary skill.

It has been reported that macrolides, such as erythromycin, clarithromycin, and azithromycin elicit pain upon parenteral administration. It has been discovered as described herein that CEM-101 and related compounds elicit low pain or no pain upon injection compared to other macrolide and ketolide antibiotics. Another embodiment is a pharmaceutical composition adapted for parenteral administration comprising the antibiotic compound CEM-101 or a related compound, as described herein, that is pain free or substantially pain free upon parenteral administration.

It has been reported that macrolides, such as clarithromycin, and especially other ketolides such as telithromycin elicit QT effects. Further, it has been reported that clarithromycin accumulated in cardiac tissue. Accordingly, though without being bound by theory, it is believed herein that such high cardiac levels may account for the QT effects, such as QT prolongation, observed with macrolides and ketolides. Without being bound by theory, it is also believed herein that such QT effects are exacerbated by the pharmacokinetics (PK) and pharmacodyanamics (PD) associated with parenteral delivery when compared to oral delivery. Such PK and PD may limit the use of clarithromycin due to the potential for higher than desired Cmax and or earlier than desired Tmax. It has been discovered as described herein that CEM-101 and related compounds elicit low QT effects or no QT effects compared to other macrolides, and especially ketolides. It has been discovered herein that CEM-101 and related compounds do not exhibit high cardiac tissue levels compared to other macrolides and ketolides. Accordingly, though without being bound by theory, it is believed herein that the low heart levels may account for the low or complete absence of QT effects, such as QT prolongation.

It also has been discovered that the parenteral administration of CEM-101 and related compounds may be rapid. Without being bound by theory, it is believed that other known macrolides and ketolides cannot be administered rapidly due to unwanted accompanying effects associated with the PK of the compounds when administered parenterally compared to the PK following oral administration. For example, it is appreciated that parenteral administration may lead to higher Cmax and/or shorter Tmax when administering other macrolides and ketolides than other delivery routes used, such as oral routes. Accordingly, side effects associated with those altered PK parameters, such as QT prolongation, and pain, may become more problematic when parenterally administering other macrolides and ketolides. Therefore, other macrolides and ketolides may be necessarily administered more slowly in an effort to balance the PK and PD associated with parenteral administration with undesired side effects such as QT effects, pain, and inflammation.

Without being bound by theory, it is believed herein that the relatively low pH of known parenteral compositions contributes to, exacerbates, or causes inflammation. Without being bound by theory, it is believed herein that the slow administration of low pH compositions may contribute to, exacerbate, or cause inflammation at the administration site due to the prolonged alteration of pH. Accordingly, current parenteral formulations of macrolides may be limited by or precluded by inflammatory side effects. It has been discovered herein that rapid infusion of parenteral compositions decreases and/or avoids inflammatory responses in the patient. However, as described herein, rapid infusion of current macrolides and ketolides is precluded by other unwanted side effects that may accompany rapid infusion.

It has further been discovered that CEM-101 and related compounds may be infused and/or administered rapidly without the accompanying unwanted side effects, such as pain and/or QT effects, and/or inflammation caused by prolonged changes in the pH at or near the site of administration. Accordingly, also described herein are compositions and methods adapted for rapid parenteral administration of CEM-101 and related compounds.

Current formulations of erythromycin and clarithromycin include lactobionic acid in a ratio of at least 1:1 to overcome the poor solubility of the macrolide. Similarly, current formulations of azithromycin include citric acid in a ratio of at least 1:1 to overcome the poor solubility of the macrolide. It has been unexpectedly found that CEM-101 and related compounds may be solubilized with substantially lower relative amounts of acidifying agents. For example, CEM-101 and related compounds for clear solutions with acidifying agents, such as tartaric acids, at ratios to CEM-101 of about 1:2, 1:5, 1:10 and the like.

As another embodiment of the invention, there is provided a method of treatment of a bacterial infection, a protozoal infection, or a disorder related to a bacterial infection or protozoal infection comprising the step of administering to a subject in need thereof a therapeutically effective amount of a pharmaceutical composition adapted for parenteral administration comprising the antibiotic compound CEM-101 or a related compound as described herein.

As another embodiment of the invention, there is provided a use of a pharmaceutical composition adapted for parenteral administration comprising the antibiotic compound CEM-101 or a related compound, as described herein, for the treatment of a bacterial infection, a protozoal infection, or a disorder related to a bacterial infection or protozoal infection.

As another embodiment of the invention, there is provided a use of a pharmaceutical composition adapted for parenteral administration comprising the antibiotic compound CEM-101 or a related compound, as described herein, for the manufacture of a medicament for the treatment of a bacterial infection, a protozoal infection, or a disorder related to a bacterial infection or protozoal infection.

As a further embodiment, a method or use described above is one wherein the subject is a mammal, a fish, a bird or a reptile. As another embodiment, there is provided a method or use wherein the subject is a mammal. As another embodiment, there is provided a method or use wherein the subject is a human.

In one illustrative embodiment, the use or method described herein is for treatment of moderate or severe community acquired pneumonia (CAP). Thus, the methods include the parenteral administration of one or more compounds described herein to a patient suffering from, or in need of relief from CAP.

In another embodiment, the use or method described herein is for treatment of severe community acquired pneumonia (CAP). One embodiment is one comprising the use or method described herein and further comprising the parenteral co-administration of CEM-101 or a related compound and another antibiotic for the treatment of severe CAP.

In another embodiment, the use or method described herein is for treatment of syphilis. In another embodiment, the use or method described herein is for treatment of gonococcal infections, such as gonococcal urethritis. It is appreciated herein that oral administration requiring multiple doses in the treatment of syphilis and/or gonococcal infections, such as gonococcal urethritis is not optimal. It is further appreciated that a parenteral administration of a composition described herein may include a single administered dose. In another embodiment, methods for treating syphilis and/or gonococcal infections, such as gonococcal urethritis are described herein, where the methods include the step of parenterally administering a composition described herein. In another embodiment, methods for treating syphilis and/or gonococcal infections, such as gonococcal urethritis are described herein, where the methods include the step of parenterally administering a single dose of a composition described herein.

A further embodiment is a method or use described herein wherein the parenteral administration comprises intravenous injection. In one embodiment, the intravenous injection is a continuous infusion. In another embodiment, the intravenous injection is a bolus injection.

A further embodiment is a method or use described herein wherein the parenteral administration comprises intramuscular injection. In one embodiment, the intramuscular injection is a continuous infusion. In another embodiment, the intramuscular injection is a bolus injection.

The following examples further illustrate specific embodiments of the invention; however, the following illustrative examples should not be interpreted in any way to limit invention.

### EXAMPLES

For any of the examples herein including CEM-101, the source of CEM-101 may be of any form or mixture thereof, including a solution, suspension, or solid. Solid forms may be an amorphous form or one or more crystalline forms, or mixtures thereof. Illustrative crystal forms of CEM-101 are described in U.S. Provisional Application No. 61/316,063.

EXAMPLE 1. Pharmaceutical Composition of CEM-101 in a Buffered Solution Containing an Antioxidant for Parenteral Administration. This example provides a process for the preparation of a 50 mg/mL IV solution having a pH of 3.7-4.2 (target 3.8) at laboratory bench scale (typically 10 to 500 mL) for administration of CEM-101 as a bolus or by infusion. The CEM-101 drug substance is protected from light when in solution and protected from oxidation by nitrogen sparging and purging. The quantities in the table below are for a scale of 1 mL.

| Materials | Grades | Functions | Percent Quantities (%w/v) | Weight Quantities for Unit Dose (mg/mL) |
|---|---|---|---|---|
| CEM-101 | - | Active | 5.0000 | 50.000 |
| 1-Thioglycerol | USP/EP | Anti-oxidant | 0.5000 | 5.000 |
| L-(+)-Tartaric Acid | USP/EP | Acidifying Agent | 0.5772 | 5.772 |
| Sodium Hydroxide | USP/EP | Alkalizing Agent | 0.0462 | 0.462 |
| Water for Injection | USP/EP | Diluent | To 100% | To 1mL |

The process comprises the following: optionally use nitrogen sparged water for injection (WFI) for the manufacturing process, and also optionally purge headspace in individual vials with nitrogen prior to crimping; weigh the required quantities of tartaric acid and sodium hydroxide into an amber volumetric flask; dispense approximately 80% of the required volume of nitrogen sparged WFI into the volumetric flask, ensure all or substantially all solid materials are completely dissolved either by sonication or stirring with a magnetic bar; ensure solution is completely or substantially clear; add the required amount of 1-thioglycerol, and ensure complete solution by stirring using a magnetic bar; add the required amount of drug substance, and ensure complete solution of the drug substance by either stirring or sonication; ensure resultant solution is clear. If the drug substance does not readily dissolve, optionally add more nitrogen sparged WFI, mix by stirring and sonication. Remove stirring bar and make up to final volume with nitrogen sparged WFI. Ensure resultant solution is clear.

Using 2 mL amber vials and Fluoretec Wester RS 13 mm Injection Stoppers for each 1 mL of the clear solution with a brown tinge, the resulting solution is either (a) filled into the amber vials, purged with nitrogen, crimped and terminally sterilized in an autoclave; or (b) aseptically filtered through a 0.2 µm pore size Fluorodyne filter, filled into the amber vials, purged with nitrogen and crimped.

EXAMPLE 2. Composition of CEM-101 in a Buffered Solution for Parenteral Administration. This example provides a process for the preparation of a 50 mg/mL IV solution having a pH of 3.7-4.2 (target 3.8) at laboratory bench scale (typically 10 to 500 mL) for administration of CEM-101 as a bolus or by infusion. The CEM-101 drug substance is protected from light when in solution and protected from oxidation by nitrogen sparging and purging. The quantities in the table below are for a scale of 1 mL.

| Materials | Grades | Functions | Percent Quantities (%w/v) | Weight Quantities for Unit Dose (mg/mL) |
|---|---|---|---|---|
| CEM-101 | - | Active | 5.0000 | 50.000 |
| L-(+)-Tartaric Acid | USP/EP | Acidifying Agent | 0.5772 | 5.772 |
| Sodium Hydroxide | USP/EP | Alkalizing Agent | 0.0462 | 0.462 |
| Water for Injection (WFI) | USP/EP | Diluent | To 100% | To 1mL |

Use nitrogen sparged water for injection (WFI) for the manufacturing process, and also purge headspace in individual vials with nitrogen prior to crimping. Weigh the required quantities of tartaric acid and sodium hydroxide into an amber volumetric flask. Dispense approximately 80% of the required volume of nitrogen sparged WFI into the volumetric flask. Ensure all solid materials are completely dissolved either by sonication or stirring with a magnetic bar. Ensure solution is completely clear. Then add the required amount of drug substance, and ensure complete solution of the drug substance by either stirring or sonication. Ensure resultant solution is clear. If the drug substance does not readily dissolve, add more nitrogen sparged WFI, mix by stirring and sonication. Remove stirring bar and make up to final volume with nitrogen sparged WFI. Ensure resultant solution is clear.

Using 2 mL amber vials and Fluoretec Wester RS 13 mm Injection Stoppers for each 1 mL of the clear solution with a brown tinge, the resulting solution is either (a) filled into the amber vials, purged with nitrogen, crimped and terminally sterilized in an autoclave; or (b) aseptically filtered through a 0.2 µm pore size Fluorodyne filter, filled into the amber vials, purged with nitrogen and crimped.

EXAMPLE 3A. Compositions of CEM-101 in an Unbuffered Solution for Parenteral Administration and Studies in 0.9% NaCl solution (∼5% w/v CEM-101). 51.38 mg CEM-101 was dissolved in 1 mL 60 mM tartaric acid pH 2.16 and allowed to mix overnight. The resultant clear solution (50.4 mg/mL by HPLC) was split into two lots by withdrawing 0.1 mL (5% initial formulation), and to the remainder was added 0.9% w/v NaCl (5% NaCl formulation). On addition of NaCl, the resultant solution went clear on mixing for an hour (53.0 mg/mL by HPLC). Thus, the inclusion of 0.9 w/v% NaCl in tartaric acid solution containing approximately 50 mg/mL CEM-101 does not have any effect on CEM-101 solubility; and an IV formulation with a drug load not exceeding 5% (equivalent to 50 mg/mL) would not lead to the precipitation of CEM-101 on addition of 0.9% NaCl under ambient conditions.

Physical stability on storage of the above formulations was assessed by storage at 4° C in a refrigerator and checking for precipitation. A sample prepared as described above for the 5% initial formulation remained in solution for at least 3 days; however, precipitation occurred in the 5% NaCl formulation after one night.

EXAMPLE 3B. Compositions of CEM-101 in an Unbuffered Solution for Parenteral Administration and Studies in 0.9% NaCl solution (∼8% w/v CEM-101). Approximately 90 mg CEM-101 was dissolved in 1 mL 60 mM tartaric acid pH 2.16 and allowed to mix overnight. The resultant clear solution (83.0 mg/mL by HPLC, saturated solubility) was split into two lots by withdrawing 0.1 mL (8% initial formulation) and to the remainder was added 0.9%w/v NaCl (8% NaCl formulation). On addition of NaCl, the resultant solution formed a white lump or ball, suggesting the precipitation of CEM-101 on addition of 0.9% w/v NaCl. The resultant solution was allowed to mix for approximately 2 hours before the concentration of CEM-101 was measured by HPLC as 3.9 mg/mL. Thus, the maximum solubility 83.0 mg (approximately 8%) seen for CEM-101 in tartaric acid is 60% higher than the target dose (50 mg/mL, 5% drug load) for the CEM-101 IV formulation.

EXAMPLE 4. Pharmaceutical Composition of CEM-101 in a Buffered Solution Containing Mannitol and an Antioxidant for Parenteral Administration.

### a. Preparation of Vehicle (also usable as Control):

Instructions which will yield a final volume of 1000 mL of the vehicle:
1. Weigh approximately 600 g of sterile water for injection, USP.
2. Add 30 g of mannitol.
3. Weigh and add 5.773 g of L(+)-tartaric acid.
4. Add 11.5 g of 1 N NaOH and mix until clear.
5. Weigh 5 g of 1-thioglycerol in a glove box under nitrogen, add to the solution, mix and measure the pH.
6. Adjust the pH to 4.2 ± 0.2 with 0.1 or 1 N NaOH or HCl.
7. QS with sterile water for injection to a final volume of 1000 mL.

The vehicle formulation can be mixed on a stir plate with a stir bar.

### b. Preparation of CEM-101 Stock Solution (5 mg/mL CEM-101):

1. Measure approximately 60 mL of the above vehicle.
2. Weigh 525 mg (500 mg corrected in this case for purity with a correction factor of 1.05) of CEM-101, add to the vehicle, mix, and measure the pH.
3. Adjust the pH to 4.2 ± 0.2 with 0.1 or 1 N NaOH or HCl.
4. QS with Control/Vehicle article formulation to a final volume of 100 mL.

The stock solution can be mixed on a stir plate with a stir bar. A glass rod and/or sonication can also be used in the preparation of the stock solution.

### c. Preparation of dosing formulations of CEM-101:

1. Measure an appropriate volume of the stock solution (5 mg/mL CEM-101).
2. Dilute the stock solution with the appropriate volume of the above vehicle to achieve the desired concentration.
3. Mix and measure the pH.

The dosing formulations can be mixed on a stir plate with a stir bar or by inversion.

Each final dosing formulation (including the control/vehicle) is filtered through a 0.22 µm PVDF filter.

EXAMPLE 5. Solubility of CEM-101 in a Range of Vehicles. Approximately 50 mg of CEM-101 is dissolved in each vehicle detailed in the table below and allowed to mix on a roller mixer for at least 48 hours. Additional CEM-101 is added to vehicles which completely solubilized the CEM-101 API within 24 hours.

Samples are allowed to mix for a total of 72 hours. Once mixed, the samples are assessed visually, centrifuged and then diluted to appropriate concentration for analysis by HPLC to determine saturated solubility.

### Saturated and Visual Solubility Results for CEM-101 in a range of Vehicles

| Sample No. | Vehicle | Saturated Solubility (mg/mL) |
|---|---|---|
| 15 | 1M Hydrochloric Acid | 100 |
| 16 | 1M Methane sulphonic Acid | 100 (pH = 0.03) |
| 17 | 1M Ascorbic Acid | 100 (pH = 1.93) |
| 19 | 1M Tartaric Acid | 100 (pH = 1.27) |

Visual solubility assessment was repeated on these vehicles to confirm initial results (see table below).

Without being bound by theory, it is believed herein that the vehicles are in situ salt forming compounds when mixed with CEM-101. Studies were conducted by weighing approximately 1000 mg of each vehicle into a number of vials. A known amount of CEM-101 was then added into each vial and the resulting mixture was mixed on a vortex prior to mixing overnight on a roller mixer.

Solubility values of 100 mg/mL were seen in all 1 Molar acid solutions (in-situ salt formers, excipients 15 - 19) investigated. pH values for resultant solutions are reported in the prior table above.

EXAMPLE 6. Characterization of Solutions for Parenteral Administration Not Containing an Antioxidant. Further studies were carried out on the following formulations of approximately 50 mg/mL, manufactured in 10 mL batch sizes, and characterized as follows:

| Formulation | Composition | Initial pH of buffer | Final pH of mixture | Visual Appearance |
|---|---|---|---|---|
| A | 50mM Tartaric Acid/NaOH Buffer + CEM-101 | 2.64 | 4.20 | Clear |
| B | 60mM Tartaric Acid + CEM-101 + 10% w/v Mannitol | 2.18 | 4.19 | Clear |
| C | 60mM Tartaric Acid + CEM-101 | 2.18 | 3.53 | Slightly Turbid |

Dilution studies were conducted using 0.9% sodium chloride solution. Dilution factors of 10 and 50 were investigated. No evidence of precipitation was observed for all the formulations investigated.

Assay analyses were conducted by using an HPLC method and are shown in the following table. Turbid samples were centrifuged prior to analysis. Total Impurities are assessed by % HPLC peak area.

| Formulation | Assay (mg/mL) | % Assay | Total Impurities |
|---|---|---|---|
| Preformulation | | | 2.34 |
| A | 47.01 | 94.01 | 1.95 |
| B | 46.02 | 92.03 | 1.89 |
| C | 47.34 | 94.68 | 1.91 |

EXAMPLE 7. Characterization of Unbuffered Formulation C Solutions with Nitrogen Sparging and with the Inclusion of an Antioxidant with or without a Chelating Agent. Development studies were conducted using Formulation C, Example C, and its corresponding placebo formulation. The table below details the composition of Formulation C active formulation and corresponding placebo.

| BATCH No: | LS1P | LS1A | LS2P | LS2A |
|---|---|---|---|---|
| MATERIALS | PLACEBO (containing EDTA + monothioglycerol) | FORMULATION C (containing added mono-thioglycerol + EDTA) | PLACEBO (containing monothioglycerol) | FORMULATION C (containing added monothioglycerol) |
| CEM-101 | - | 5%w/w | - | 5%w/w |
| Monothioglycerol | 1%w/w | 1%w/w | 1%w/w | 1%w/w |
| EDTA | 0.025%w/w | 0.025%w/w | - | - |
| 60 mM Tartaric Acid | To 100% | To 100% | To 100% | To 100% |

The solutions above were prepared by first dissolving the stabilizers in the vehicle (60 mM Tartaric acid) which was pre-prepared in nitrogen sparged water. Once dissolved, the CEM-101 API was then added slowly and allowed to mix until a complete solution was achieved.

Formulations prepared were then filled into vials, crimped with an aluminium cap containing a PTFE septum and then autoclaved. Each vial was nitrogen purged prior to crimping.

HPLC analyses were performed on both autoclaved and non-autoclaved samples. Refer to results in the table below for summary data on assay and impurities.

### Assay and Impurity Levels.

| Formulation | Conditions | Sample Identity | Total Impurities | Assay (%) |
|---|---|---|---|---|
| STD 0.5 mg/mL | - | - | 1.867 | - |
| LS1A | Non-Autoclaved | LS1ANA-1 | 1.592 | 102.97 |
| | | LS1ANA-2 | 1.426 | 104.28 |
| | Autoclaved | LS1AA-1 | 1.335 | 103.17 |
| | | | | |
| LS2A | Non-Autoclaved | LS2ANA-1 | 1.600 | 100.45 |
| | | LS2ANA-2 | 1.620 | 100.53 |
| | Autoclaved | LS2AA-1 | 1.422 | 107.67 |

Assay results seen for Formulation C containing various combinations of stabilizers were approximately 100%. Unknown impurity products seen for both formulations were similar to those seen for the active compound except for impurity peaks on autoclaving seen at RRT0.98.

Total levels of unknown impurity products seen for both formulations were lower than those seen for the active compound. This data suggest both formulations investigated were as stable as the active compound pre- and post- autoclave cycle.

Studies from previous experiments investigating a range of stabilizers showed impurity levels as high as 4% with assay values as low as 88%. The data detailed in this example shows an improvement in the control of degradation products seem with these formulations and nitrogen sparging.

EXAMPLE 8. Determination of Compatibility of Excipients for Lyophilized Pharmaceutical Composition. Excipient compatibility is assessed using high sensitivity multi-cell differential scanning calorimetry (HS-MCDSC). The high sensitivity multi-cell differential scanning calorimeter is an analytical instrument designed to measure heat capacity and changes in heat capacity as a function of temperature.

HS-MCDSC is useful for compatibility assessment because it is sensitive enough to allow the analysis of samples non-destructively (that is, it does not cause any extra degradation than that which would have occurred upon storage). This example details the results of the compatibility study on drug/excipients mixtures which were found to be acceptable using HS-MCDSC.

In HS-MCDSC, the heat-flow to or from a sample (power,φ, in µW) is measured as a function of time (s). Integration of the power-time data gives the change in heat content (enthalpy, Δ*H*, in µJ). Since heat is universal, virtually any sample can be studied using the technique as long as a representative sample can fit into the cell, and the heat-flows of all the processes, chemical or physical, occurring within the sample are recorded. This allows the investigation of complex systems that would normally fall outside the scope of traditional analytical techniques, but often results in complex data that are difficult to interpret. It also means that sample preparation is paramount; careless sample preparation may result in erroneous heat-flows which will subsequently prevent accurate data interpretation.

In HS-MCDSC any heat produced or absorbed by a sample is, ideally, completely exchanged with a surrounding heat-sink, maintaining the sample at a wide temperature range. Usually a reference cell (cell #4) is loaded with an inert material of similar heat capacity, and of a similar quantity to the sample, and data are obtained as a differential response between sample and reference. Consequently, most of the noise arising from temperature fluctuations is removed when the blank data (running empty cells prior to the real sample experiment) are subtracted from the sample data.

Stability (of an individual component) is usually assessed by loading a sample of the material into a cell and measuring the heat-flow as a function of time. The instrument should rapidly detect any degradation processes occurring, and the absence of any heat responses gives confidence that the system under investigation is stable.

A similar approach can be adopted for testing binary mixtures of the active pharmaceutical ingredient (API) and excipients. The thermal behaviour, over a wide temperature range, of the active alone and the excipients alone, are recorded and compared with that determined for a binary drug-excipients mixture. Any unexpected power change recorded in the drug-excipient mixture indicates a possible interaction between the two components.

Calorimetric data were recorded using a high sensitivity MCDSC (CSC Model 4100 MCDSC, USA). Samples were run using API alone (ca. 50 mg), excipients alone (ca. 50 mg) and API - Excipient binary pairs (ca. 25 mg:25 mg, 1:1 ratio). Samples were weighed directly into the cells which were sealed with the metal caps provided and a rubber sealing disc to ensure an air-tight seal. Experiments were programmed as follows:
Holding at 30 °C for 10800 seconds (s) before moving into the next temperature.
Heating: 30 °C to 40 °C at a scan rate of 0.5 °C/min.
Holding: Isothermally at 40 °C for 10800 s.
Heating: 40 °C to 50 °C at a scan rate of 0.5 °C/min.
Holding: Isothermally at 50 °C for 10800 s
Heating: 50 °C to 60 °C at a scan rate of 0.5°C/min.
Holding: Isothermally at 60 °C for 10800 s.
Heating: 60 °C to 70 °C at a scan rate of 0.5 °C/min.
Holding: Isothermally at 70 °C for 10800 s.
Heating: 70 °C to 80 °C at a scan rate of 0.5 °C/min.
Holding: Isothermally at 80 °C for 10800 s.
Cooling: Cool back to 30 °C from 80 °C.

The MCDSC consists of four cells contained in a loading chamber. The cells 1, 2 and 3 were filled with samples, while the 4^{th} cell is always left empty and run as a blank. Prior to analysis, cells 1 to 3 were run empty with no samples to record the blank results which were then subtracted from subsequent results obtained from samples analysed. Data were recorded every 10 s using the dedicated software package CpCalc. Data analysis was performed using CpCalc and Microsoft Excel.

The calorimeter records all the events occurring in each of the sample cell. The approach adopted here was to calculate the power-time data obtained for the active and excipients alone. The calculated data is then used to create an 'expected' theoretical trace as an average of the signals coming from both API and excipients at a mixture of 1:1 ratio. These data were then compared with those obtained for the actual binary mixtures. Any significant differences in the power-time data (different shape or intensity in their heat flow response over the experimental timeframe) suggest an incompatibility. MC-DSC indicated that mannitol, glycine, and sucrose were compatible with CEM-101 at a ratio of 1:1.

EXAMPLE 9. Characterization of properties of excipients for freeze drying to prepare lyophilized pharmaceutical composition. Following the above results, lyophilisation studies were conducted by initially assessing the solubility of each excipient in CEM - 101 IV solutions (50 mg/mL, prepared in a manner similar to that described in Example 2, above) as follows:
1 mL of CEM-101 IV solution was dispensed into in a clear glass vial, followed by the careful addition of small amounts of the excipient under investigation (either 50 or 100 mg). The solution in the vials were mixed using a vortex mixer and sonicated until a clear solution was observed. The final data is shown below, and reflects the maximum possible amount of each excipient used in the freeze dried formulations. Subsequently, all clear vials were frozen at -25 °C for 2 h. The caps were then removed and the vials were covered with a small tissue paper and placed in the freeze drier for 24 h (in the dark). The cakes were then inspected and the following visual observations detailed in the table below were made.

### Summary of the Cakes obtained during the Freeze Drying Experiments and MC-DSC Analysis

| Excipient | Conc. (mg/mL) | Ratio of API:Excipient (assuming 50mg API) | Visual observation of the cake | MC-DSC Results (No Interaction/ Interaction) |
|---|---|---|---|---|
| Mannitol | 200 | 1:4 | Good solid cake - with uniform distribution of the constituents | No Interaction |
| Isomalt | 600 | 1:12 | Good solid cake - with uniform distribution of the constituents | Interaction |
| Glycine | 200 | 1:4 | Good solid cake - with uniform distribution of the constituents | No Interaction |
| Sucrose | 500 | 1:10 | Meltback of the cake | No Interaction |
| Trehalose | 700 | 1:14 | Poor cake - puffing due to the incomplete freezing of the matrix | Interaction |

Based on the results above, three excipients with no interaction with the API were selected and solutions containing various ratio combinations of the API and excipient were prepared. These solutions were then frozen at -25 °C, then lyophilised for 24 h, in the dark. The resultant cakes were then examined visually to determine the integrity of the plugs. The results are shown in the table below.

### Visual Observation of Lyophilizates

| Excipient | API:Excipient Ratio | Visual observation |
|---|---|---|
| Mannitol | 1:1 | Good solid cake- with uniform distribution of constituents |
| | 1:2 | Good solid cake- with uniform distribution of constituents |
| | 1:3 | Good solid cake- with uniform distribution of constituents |
| | 1:4 | Good solid cake- with uniform distribution of constituents |
| Glycine | 1:1 | Good solid cake- with uniform distribution of constituents |
| | 1:2 | Good solid cake- with uniform distribution of constituents |
| | 1:3 | Good solid cake- with uniform distribution of constituents |
| | 1:4 | Good solid cake- with uniform distribution of constituents |
| Sucrose | 1:1 | Good solid cake- with uniform distribution of constituents |
| | 1:2 | Good solid cake- with uniform distribution of constituents |
| | 1:3 | Good solid cake- with uniform distribution of constituents |
| | 1:4 | Good solid cake- with uniform distribution of constituents |

Cakes were stored at 5°C, 25°C/60%RH and 40°C/75%RH. Samples were evaluated at 7, 13 and 28 days. In all cases, the assay remained unchanged for all three bulking agents. The mannitol and glycine formulation cakes remained white throughout the study. Some cake yellowing was observed in a portion of samples of the sucrose formulations.

EXAMPLE 10. Pharmacokinetics (PK) in monkeys following IV administration. A pharmaceutical composition adapted for parenteral administration comprising the antibiotic compound CEM-101, prepared as described herein is administered intravenously daily at 5, 12.5, and 25 mg/kg/d to male (M) and female (F) Cynomolgus monkeys (Macaca fasicularis, 2-5 kg, young adult, acclimated) for 14 days or 28 days, and compared to vehicle treated animals. The plasma level of CEM-101 and toxicokinetics at 0-24 hours is measured (venipuncture, predose, then immediately following completion of infusion at 0, 0.5, 1, 4, 8, and 24 hours). Animal body weight is also periodically measured.

The high dose is selected to provide a multiple of an illustrative human Cmax exposure of 1 µg/mL and the low dose is selected to provide an illustrative human exposure at or slightly above a typical dose amount. Total dose volume is 15 mL/kg, and intravenous infusion is carried out over about 90 minutes (10 mL/kg/h) via disposable indwelling catheter into one of the brachial and/or saphenous veins. Vein or vein location is changed at least every seven days. Control animals receive vehicle alone. An illustrative formulation is CEM-101 in the following vehicle comprising or consisting essentially of L(+)-tartaric acid, D-mannitol, SWFI, 1N HaOH, and optionally 1-thioglycerol, as described herein.

Following dosing, a portion of the animals are immediately euthanized for necropsy evaluation. A "recovery" group is observed for an additional 28 days without dosing, then euthanized for necropsy evaluation.

EXAMPLE 11. Pharmacokinetics (PK) in dogs following IV administration. A pharmaceutical composition adapted for parenteral administration comprising the antibiotic compound CEM-101, prepared as described herein is administered intravenously daily at 5, 10 and 15 mg/kg/d to male (M) and female (F) Beagle dogs (Canis familiaris, 7-10 kg. 5-7 months, 2-3 weeks acclimation) for 14 days or 28 days, and compared to vehicle treated animals. The plasma level of CEM-101 and toxicokinetics at 0-24 hours is measured (jugular venipuncture, predose, then immediately following completion of infusion at 0, 0.5, 1, 4, 8, and 24 hours). Animal body weight is also periodically measured.

The high dose is selected to provide a multiple of an illustrative human Cmax exposure of 1 µg/mL and the low dose is selected to provide an illustrative human exposure at or slightly above a typical dose amount. Total dose volume is 15 mL/kg, and intravenous infusion is carried out over about 90 minutes (10 mL/kg/h) via disposable indwelling catheter into one of the cephalic and/or saphenous veins. Vein or vein location is changed at least every seven days. Control animals receive vehicle alone. An illustrative formulation is CEM-101 in the following vehicle comprising or consisting essentially of L(+)-tartaric acid, D-mannitol, SWFI, 1N HaOH, and optionally 1-thioglycerol, as described herein.

Following dosing, a portion of the animals are immediately euthanized for necropsy evaluation. A "recovery" group is observed for an additional 28 days without dosing, then euthanized for necropsy evaluation.

It is appreciated that both monkeys and dogs elicit particular sensitivity to pain, and therefore may be observed for pain response upon administration of the test articles. In each case, no reaction by the animal was observed following the IV administration of CEM-101, indicating that the administration was pain free or substantially pain free.

## Claims

1. A pharmaceutical composition adapted for parenteral administration comprising solithromycin or a pharmaceutically acceptable salt thereof; and an acidifying agent comprising a tartaric acid or citric acid, or a combination thereof; where the composition is capable of reconstitution in one or more aqueous diluents.

2. A lyophilized pharmaceutical composition, adapted for dilution to a pharmaceutical composition for parenteral administration, comprising solithromycin or a pharmaceutically acceptable salt thereof, an acidifying agent comprising a tartaric acid or citric acid, or a combination thereof, an alkalizing agent, and at least one additional excipient.

3. A kit, comprising the lyophilized pharmaceutical composition as described in claim 2 and further comprising a vehicle for dilution of the pharmaceutical composition, and instructions for said dilution of the pharmaceutical composition.

4. The composition of claim 1 further comprising one or more bulking agents.

5. The composition of claim 4 wherein at least one of the bulking agents is selected from the group consisting of mannitol, sucrose, and glycine.

6. The composition of claim 4 wherein at least one of the bulking agents is mannitol.

7. The composition of any one of claims 4 to 6 wherein the ratio of bulking agent to solithromycin is in the range from 1:2 to 10:1, preferably in the range from 1:1 to 5:1; preferably in the range from 1:1 to 4:1.

8. The composition of any one of claims 1 to 7 wherein the ratio of solithromycin to acidifying agent is in the range from 100:1 to 2:1; preferably in the range from 20:1 to 5:1; preferably in the range from 10:1 to 5:1.

9. The composition of claim 8 wherein the acidifying agent is L tartaric acid.

10. The composition of claim 9 wherein the ratio of acidifying agent to solithromycin is in the range from 0.01:1 to 0.5:1.

11. The composition of any one of claims 1 to 10 reconstitutable to a concentration of solithromycin of at least about 30 mg/mL.

12. The composition of any one of claims 1 to 10 reconstitutable to a concentration of solithromycin of at least about 50 mg/mL.

13. A pharmaceutical composition for use in the treatment of a bacterial infection, a protozoal infection, or a disorder related to a bacterial infection or protozoal infection in a patient, the pharmaceutical composition comprising a therapeutically effective amount of the composition of any one of claims 1 to 12.

14. The pharmaceutical composition of claim 13 wherein the bacterial infection is community acquired pneumonia.

15. The pharmaceutical composition of claim 13 wherein the bacterial infection is gonococcal urethritis.

## Patentansprüche

1. Zur parenteralen Verabreichung geeignete pharmazeutische Zusammensetzung, enthaltend Solithromycin oder ein pharmazeutisch zulässiges Salz davon und ein Säuerungsmittel, umfassend Weinsäure oder Zitronensäure oder eine Kombination aus diesen, wobei die Zusammensetzung in einem oder mehreren wässrigen Verdünnern rekonstituiert werden kann.

2. Lyophilisierte pharmazeutische Zusammensetzung, geeignet zum Verdünnen zu einer pharmazeutischen Zusammensetzung für die parenterale Verabreichung, enthaltend Solithromycin oder ein pharmazeutisch zulässiges Salz davon und ein Säuerungsmittel, umfassend Weinsäure oder Zitronensäure oder eine Kombination aus diesen, ein Alkalisiermittel und mindestens einen Hilfsstoff.

3. Set, umfassend die lyophilisierte pharmazeutische Zusammensetzung nach Anspruch 2, ferner umfassend ein Mittel zum Verdünnen der pharmazeutischen Zusammensetzung sowie Anweisungen für dieses Verdünnen der pharmazeutischen Zusammensetzung.

4. Zusammensetzung nach Anspruch 1, ferner einen oder mehrere Füllstoffe umfassend.

5. Zusammensetzung nach Anspruch 4, wobei mindestens ein Füllstoff aus der aus Mannit, Saccharose und Glycin bestehenden Gruppe ausgewählt ist.

6. Zusammensetzung nach Anspruch 4, wobei der mindestens eine Füllstoff Mannit ist.

7. Zusammensetzung nach einem der Ansprüche 4 bis 6, wobei das Verhältnis von Füllstoff zu Solithromycin im Bereich 1:2 bis 10:1 liegt, vorzugsweise im Bereich 1:1 bis 5:1, bevorzugt im Bereich 1:1 bis 4:1.

8. Zusammensetzung nach einem der Ansprüche 1 bis 7, wobei das Verhältnis von Solithromycin zu Säuerungsmittel im Bereich von 100:1 bis 2:1 liegt, vorzugsweise im Bereich 20:1 bis 5:1, bevorzugt im Bereich 10:1 bis 5:1.

9. Zusammensetzung nach Anspruch 8, wobei das Säuerungsmittel Weinsäure ist.

10. Zusammensetzung nach Anspruch 9, wobei das Verhältnis von Säuerungsmittel zu Solithromycin im Bereich 0,01:1 bis 0,5:1 liegt.

11. Zusammensetzung nach einem der Ansprüche 1 bis 10, das zu einer Solithromycinkonzentration von mindestens 30 mg/l rekonstituiert werden kann.

12. Zusammensetzung nach einem der Ansprüche 1 bis 10, das zu einer Solithromycinkonzentration von mindestens 50 mg/l rekonstituiert werden kann.

13. Pharmazeutische Zusammensetzung zur Verwendung bei der Behandlung einer bakteriellen Infektion, einer Protozoeninfektion oder einer mit bakterieller Infektion oder Protozoeninfektion zusammenhängenden Störung eines Patienten, wobei die pharmazeutische Zusammensetzung eine therapeutisch wirksame Menge der Zusammensetzung nach einem der Ansprüche 1 bis 12 enthält.

14. Pharmazeutische Zusammensetzung nach Anspruch 13, wobei die bakterielle Infektion ambulant erworbene Lungenentzündung ist.

15. Pharmazeutische Zusammensetzung nach Anspruch 13, wobei die bakterielle Infektion Harnröhrenentzündung durch Gonokokken ist.

## Revendications

1. Composition pharmaceutique destinée à être administrée par voie parentérale renfermant de la solithromycine ou un sel pharmaceutiquement acceptable de celle-ci et un agent acidifiant renfermant de l'acide tartrique ou de l'acide citrique ou une combinaison de ces acides, la composition étant susceptible d'être reconstituée dans un ou plusieurs diluants aqueux.

2. Composition pharmaceutique destinée à être diluée de façon à obtenir une composition pharmaceutique destinée à l'administration par voie parentérale renfermant de la solithromycine ou un sel pharmaceutiquement acceptable de celle-ci, un agent acidifiant renfermant de l'acide tartrique de l'acide citrique ou une combinaison de ces acides, un agent d'alcalinisation et au moins un excipient additionnel.

3. Kit renfermant une composition pharmaceutique lyophilisée conforme à la revendication 1, ainsi qu'un véhicule pour permettre la dilution de cette composition pharmaceutique et des instructions pour effectuer cette dilution de la composition pharmaceutique.

4. Composition conforme à la revendication 1,
comprenant en outre au moins un agent gonflant.

5. Composition conforme à la revendication 4,
dans laquelle l'agent gonflant est choisi dans le groupe formé par le mannitol, le sucrose et la glycine.

6. Composition conforme à la revendication 4,
dans laquelle l'agent gonflant est le mannitol.

7. Composition conforme à l'une quelconque des revendications 4 à 6, dans laquelle le rapport de l'agent gonflant à la solithromycine est situé dans la plage comprise entre 1:2 et 10:1, de préférence dans la plage comprise entre 1:1 et 5:1 et de façon plus préférentielle, dans la plage comprise entre 1:1 et 4:1.

8. Composition conforme à l'une quelconque des revendications 1 à 7, dans laquelle le rapport de la solithromycine à l'agent acidifiant est situé dans la plage comprise entre 100:1 et 2:1, de préférence dans la plage comprise entre 20:1 et 5:1 et de façon plus préférentielle dans la plage comprise entre 10:1 et 5:1.

9. Composition conforme à la revendication 8, dans laquelle l'agent acidifiant est l'acide L tartrique.

10. Composition conforme à la revendication 9, dans laquelle le rapport de l'agent acidifiant à la solithromycine est situé dans la plage comprise entre 0,01:1 et 0,5:1.

11. Composition conforme à l'une quelconque des revendications 1 à 10, pouvant être reconstituée pour obtenir une concentration en solithromycine d'au moins environ 30 mg/mL.

12. Composition conforme à l'une quelconque des revendications 1 à 10, pouvant être reconstituée pour obtenir une concentration en solithromycine d'au moins environ 50 mg/mL.

13. Composition pharmaceutique destinée à être utilisée pour le traitement d'une infection par des bactéries, d'une infection par des protozoaires ou d'une infection liée à une infection par des bactéries ou à une infection par des protozoaires chez un patient, cette composition pharmaceutique renfermant une quantité thérapeutiquement efficace de la composition conforme à l'une quelconque des revendications 1 à 12.

14. Composition pharmaceutique conforme à la revendication 131, dans laquelle l'infection par des bactéries est la pneumonie communautaire.

15. Composition pharmaceutique conforme à la revendication 13, dans laquelle l'infection par des bactéries est l'urétrite gonococcique.
